# EUROPEAN PATENT APPLICATION

(11) **EP 4 844 988 A1**
(43) Date of publication of application: **25.11.2026**
(21) Application number: 24926624.8
(22) Date of filing: 18.07.2024
(51) Int. Cl.: H02K 1/18, H02K 1/28, H02K 15/02, A61M 60/40

(54) **CORELESS MOTOR HAVING PROTECTIVE SLEEVE AND POTTING METHOD THEREFOR**

(30) Priority: 26.02.2024 CN 202410205840
(71) Applicant: Suzhou Hearthill Medical Technology Co., Ltd., Suzhou, Jiangsu 215000 (CN)
(72) Inventor: HAN, Liwei, Suzhou, Jiangsu 215000 (CN); GENG, Wenji, Suzhou, Jiangsu 215000 (CN); ZHENG, Siyuan, Suzhou, Jiangsu 215000 (CN); YAN, Xiaoshen, Suzhou, Jiangsu 215000 (CN)
(74) Representative: Dai, Simin
(86) International application number: PCT/CN2024/106080
(87) International publication number: WO 2025/179755

(57) **Abstract**

The present application provides a coreless motor having protective sleeves and a method of potting same. The coreless motor having protective sleeves includes a stator assembly, a rotor assembly and a motor housing. The stator assembly is disposed in the motor housing and defines an axially extending through-cavity. The rotor assembly is inserted through the cavity. The motor housing is fitted over an outer wall of the stator assembly, and opposite ends of the motor housing extend beyond opposite ends of the stator assembly in an axial direction. A stator protective sleeve is disposed on an inner wall of the stator assembly, and opposite ends of the stator protective sleeve extend beyond the opposite ends of the stator assembly in the axial direction. Stator encapsulation layers are disposed at the opposite ends of the stator assembly. The stator protective sleeve, the motor housing and the stator encapsulation layers hermetically isolate the stator assembly. The rotor assembly comprises a permanent magnet and a rotor shaft, and the rotor shaft extends through a center of the permanent magnet. A rotor protective sleeve is disposed on an outer wall of the permanent magnet, and opposite ends of the rotor protective sleeve extend beyond opposite ends of the permanent magnet in the axial direction. Rotor encapsulation layers are disposed at end portions of the permanent magnet. The rotor protective sleeve and the rotor encapsulation layers hermetically isolate the permanent magnet. The present application achieves protection for non-biocompatible materials of the motor. The present application achieves protection for non-biocompatible materials of the motor.

## Description

### Cross-Reference to Related Applications

This patent application claims priority to Chinese Patent Application No. 202410205840.5, filed on February 26, 2024, entitled "CORELESS MOTOR HAVING PROTECTIVE SLEEVES AND METHOD OF POTTING SAME", which is incorporated herein by reference in its entirety.

### Technical Field

The present application relates to the technical field of coreless motors, and in particular, to a coreless motor having protective sleeves and a method of potting same.

### Background Art

Due to their compact size, high efficiency and stable operation, coreless motors are often used in medical devices. In addition to the above characteristics, biocompatibility and safety are also essential requirements for medical devices. Therefore, full consideration must be given to material selection and isolation protection of the motor to cope with various unexpected conditions when the motor operates inside the human body. Under such operation requirements, it is critical to design a motor structure with reliable structure and robust sealing performance, and enhanced electromagnetic performance and output torque.

At present, common isolation protection methods for implantable grade coreless motors include polymer film coating or potting, which coat non-biocompatible parts of the motor such as iron cores, coils and permanent magnets to provide a barrier against blood and prevent oxidation and delamination of metal materials, which could otherwise enter the bloodstream and cause bodily contamination. However, both polymer film coating and potting have low reliability, and inevitably suffer from low yield rate and risk of delamination under impact, vibration and alternating damp heat conditions. There is a need for a protective structure with high reliability to ensure protection of non-biocompatible materials of the motor.

Accordingly, it is necessary to design a coreless motor having protective sleeves and a method of potting same to solve the above problems.

### Summary of the Invention

The technical problem to be solved by the embodiments of the present application is to provide protective sleeves for protecting a stator assembly and a rotor assembly and achieve sealing and isolation through encapsulation layers, so as to realize protection for non-biocompatible materials of the motor.

To solve the above technical problem, an embodiment of the present application provides a coreless motor having protective sleeves and a method of potting same. The coreless motor having protective sleeves includes a stator assembly, a rotor assembly and a motor housing; the stator assembly being disposed in the motor housing and defining an axially extending through-cavity, the rotor assembly being rotatably disposed in the cavity, where the motor housing is fitted over an outer wall of the stator assembly, and opposite ends of the motor housing extend axially beyond opposite ends of the stator assembly; a stator protective sleeve is disposed on an inner wall of the stator assembly, and opposite ends of the stator protective sleeve extend axially beyond the opposite ends of the stator assembly; stator encapsulation layers are disposed at the opposite ends of the stator assembly, and the stator protective sleeve, the motor housing and the stator encapsulation layers hermetically isolate the stator assembly; the rotor assembly includes a permanent magnet and a rotor shaft, the rotor shaft extends through a center of the permanent magnet; a rotor protective sleeve is disposed on an outer wall of the permanent magnet, and opposite ends of the rotor protective sleeve extend axially beyond opposite ends of the permanent magnet; rotor encapsulation layers are disposed at end portions of the permanent magnet, and the rotor protective sleeve and the rotor encapsulation layers hermetically isolate the permanent magnet.

Optionally, the stator protective sleeve and the rotor protective sleeve are each made of a biocompatible metal material, and the biocompatible metal material includes 316 stainless steel, 317 stainless steel, titanium alloy, cobalt alloy, gold, silver, platinum, and shape memory alloy. Optionally, the stator protective sleeve and the rotor protective sleeve each have a cylindrical tubular structure, and are each formed by welding a thin metal sheet made of the biocompatible metal material, and the thin metal sheet has a thickness of 0.01 mm to 0.05 mm.

Optionally, the stator encapsulation layers and the rotor encapsulation layers are each formed by potting with an adhesive.

Optionally, an outer diameter of the stator protective sleeve is smaller than an inner diameter of the stator assembly, a minimum difference between the outer diameter of the stator protective sleeve and the inner diameter of the stator assembly is 0.02 mm; the stator protective sleeve is disposed coaxially with the stator assembly, such that a minimum clearance formed between the stator protective sleeve and the stator assembly is 0.01 mm; and the stator protective sleeve and the stator assembly are bonded by an adhesive coated on an outer wall of the stator protective sleeve. Optionally, a difference between a length of the stator protective sleeve and a length of the stator assembly is 1 mm to 2 mm; a distance between an end portion of the stator protective sleeve and an end portion of the stator assembly on a same side is 0.5 mm to 1 mm; and the stator encapsulation layer is disposed between the end portion of the stator protective sleeve and the end portion of the stator assembly on the same side.

Optionally, an inner diameter of the rotor protective sleeve is larger than an outer diameter of the permanent magnet, a minimum difference between the inner diameter of the rotor protective sleeve and the outer diameter of the permanent magnet is 0.02 mm; the rotor protective sleeve is disposed coaxially with the permanent magnet, such that a minimum clearance formed between the rotor protective sleeve and the permanent magnet is 0.01 mm; and the rotor protective sleeve and the permanent magnet are bonded by an adhesive coated on an inner wall of the rotor protective sleeve. Optionally, a difference between a length of the rotor protective sleeve and a length of the permanent magnet is 1 mm to 2 mm; a distance between an end portion of the rotor protective sleeve and an end portion of the permanent magnet on a same side is 0.5 mm to 1 mm; and the rotor encapsulation layer is disposed between the end portion of the rotor protective sleeve and the end portion of the permanent magnet on the same side.

Optionally, an outer diameter of the stator assembly is equal to an inner diameter of the motor housing, and the stator assembly is connected to the motor housing via a clearance fit.

Based on the same inventive concept, an embodiment of the present application further provides a potting method for a coreless motor having protective sleeves. The coreless motor having protective sleeves includes a stator assembly, a rotor assembly and a motor housing; the stator assembly being disposed in the motor housing and defining an axially extending through-cavity, the rotor assembly is rotatably inserted through the cavity, and the rotor assembly including a permanent magnet and a rotor shaft, the rotor shaft extending through a center of the permanent magnet; where the method includes the steps of:
providing the stator assembly and a stator protective sleeve, fitting the stator protective sleeve over an inner wall of the stator assembly, and controlling opposite ends of the stator protective sleeve to extend axially beyond opposite ends of the stator assembly, with the opposite ends of the stator protective sleeve being equidistant from the opposite ends of the stator assembly in the axial direction, respectively;
providing the motor housing, fitting the motor housing over an outer wall of the stator assembly with the stator protective sleeve mounted thereon, and controlling opposite ends of the motor housing to extend axially beyond the opposite ends of the stator assembly, such that a stator potting space is defined by an outer wall of the stator protective sleeve, an inner wall of the motor housing, an end face of the stator assembly and an end face of the stator protective sleeve;
dispensing an adhesive into the stator potting space to form a stator encapsulation layer, so as to hermetically isolate the stator assembly;
providing the rotor assembly and a rotor protective sleeve, fitting the rotor protective sleeve over an outer wall of the permanent magnet, and controlling opposite ends of the rotor protective sleeve to extend axially beyond opposite ends of the permanent magnet, with the opposite ends of the rotor protective sleeve being equidistant from the opposite ends of the permanent magnet in the axial direction, respectively, such that a rotor potting space is defined by an outer wall of the rotor shaft, an inner wall of the rotor protective sleeve, an end face of the permanent magnet and an end face of the rotor protective sleeve; and
dispensing an adhesive into the rotor potting space to form a rotor encapsulation layer, so as to hermetically isolate the permanent magnet.

Optionally, an outer diameter of the stator protective sleeve is smaller than an inner diameter of the stator assembly, the stator protective sleeve is disposed coaxially with the stator assembly, such that a minimum difference between the outer diameter of the stator protective sleeve and the inner diameter of the stator assembly is 0.02 mm, and a minimum clearance formed between the stator protective sleeve and the stator assembly is 0.01 mm; the method further including the steps of:
coating an outer wall of the stator protective sleeve with an adhesive; and
fitting the stator protective sleeve over the inner wall of the stator assembly, so as to bond the stator protective sleeve and the stator assembly into one piece.

Optionally, an inner diameter of the rotor protective sleeve is larger than an outer diameter of the permanent magnet, the rotor protective sleeve is disposed coaxially with the permanent magnet, such that a minimum difference between the inner diameter of the rotor protective sleeve and the outer diameter of the permanent magnet is 0.02 mm, and a minimum clearance formed between the rotor protective sleeve and the permanent magnet is 0.01 mm; the method further including the steps of:
coating an inner wall of the rotor protective sleeve with an adhesive; and
fitting the rotor protective sleeve over the outer wall of the permanent magnet, so as to bond the rotor protective sleeve and the rotor assembly into one piece.

Compared with the prior art, the technical solutions of the embodiments of the present application have the following beneficial effects.

For example, in the coreless motor having protective sleeves and the method of potting same according to the present application, protective sleeves are used to protect the stator assembly and the rotor assembly, and sealing and isolation are realized by dispensing adhesive at end portions to form encapsulation layers, thereby achieving reliable protection for non-biocompatible materials of the motor. Especially when water enters the motor, the encapsulation layers are not easy to delaminate under water pressure scouring, with high reliability.

For another example, the protective sleeves are processed from thin metal sheets, usually titanium alloy sheets. Titanium alloy has good biocompatibility, high strength and stable structure, which is conducive to providing stable and reliable protection. Titanium alloy has low electrical conductivity, which can effectively filter temporal and spatial harmonics in the stator magnetic field, reduce eddy current loss on the rotor surface, reduce heat generation of the rotor, improve motor efficiency, and ensure the safety of motor operation. The welding process is simple and easy to manufacture, and the small thickness of the thin metal sheet does not affect the overall size of the motor.

For another example, the protective sleeves extend beyond both ends of the stator assembly or the permanent magnet of the rotor assembly to form potting spaces, which facilitates dispensing of the encapsulation layers to achieve sealing, and prevents the encapsulation layers from delaminating due to vibration during motor operation.

### Brief Description of the Drawings

FIG. 1 is a cross-sectional view of a coreless motor having protective sleeves according to an embodiment of the present application;
FIG. 2 is a schematic diagram illustrating installation of a stator assembly and a stator protective sleeve according to an embodiment of the present application;
FIG. 3 is a schematic structural diagram illustrating a motor housing, a stator assembly and a stator protective sleeve according to an embodiment of the present application;
FIG. 4 is an enlarged view of portion A in FIG. 3;
FIG. 5 is a schematic structural diagram illustrating a rotor assembly and a rotor protective sleeve according to an embodiment of the present application;
FIG. 6 is an enlarged view of portion B in FIG. 5;
FIG. 7 is a schematic structural diagram illustrating a stator protective sleeve or a rotor protective sleeve according to an embodiment of the present application;
FIG. 8 is a schematic structural diagram illustrating a thin metal sheet according to an embodiment of the present application;
FIG. 9 is a schematic structural diagram illustrating another thin metal sheet according to an embodiment of the present application;
FIG. 10 is a schematic model diagram illustrating a coreless motor without a protective sleeve in the prior art;
FIG. 11 is a schematic model diagram illustrating a coreless motor having protective sleeves according to an embodiment of the present application;
FIG. 12 is an FFT diagram illustrating back EMF of a coreless motor without a protective sleeve in the prior art;
FIG. 13 is an FFT diagram illustrating back EMF of a coreless motor having protective sleeves according to an embodiment of the present application;
FIG. 14 is a torque diagram illustrating a coreless motor without a protective sleeve in the prior art;
FIG. 15 is a torque diagram illustrating a coreless motor having protective sleeves according to an embodiment of the present application;
FIG. 16 is a cogging torque diagram illustrating a coreless motor without a protective sleeve in the prior art; and
FIG. 17 is a torque diagram illustrating a coreless motor having protective sleeves according to an embodiment of the present application.

In the drawings:
1. rotor protective sleeve;
2. stator protective sleeve;
3. rotor assembly, 31. rotor shaft, 32. permanent magnet;
4. stator assembly, 41. winding coil, 42. iron core;
5. motor housing;
6. rotor encapsulation layer;
7. stator encapsulation layer;
8. front end cover;
9. rear end cover; and
10. connection cover.

### Detailed Description of the Invention

To make the objectives, features and advantages of the present application more apparent and understandable, specific embodiments of the present application will be described in detail below with reference to the accompanying drawings. It is to be understood that the specific embodiments described below are only used to explain the present application, but not to limit the present application. Moreover, the same or similar reference numerals may be used in the drawings to refer to the same or similar elements in different embodiments, and descriptions of the same or similar elements in different embodiments and descriptions of prior art elements, features and effects may be omitted.

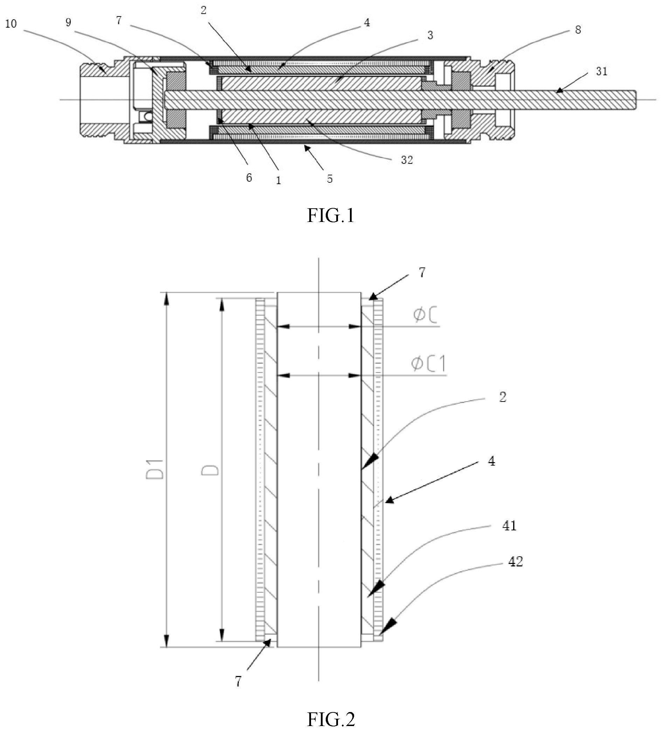

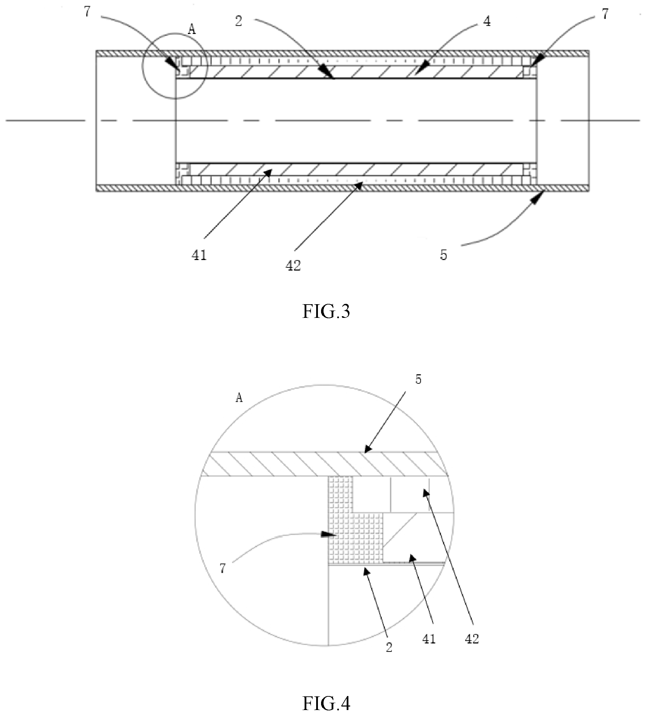

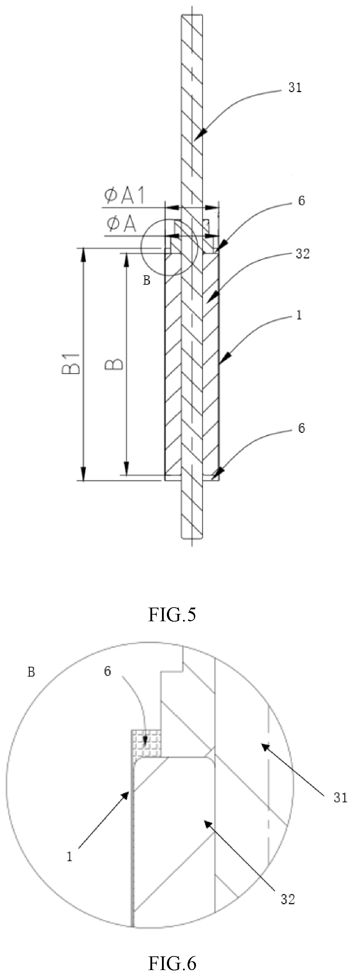

Referring to FIGS. 1 to 9, an embodiment of the present application provides a coreless motor having protective sleeves.

Specifically, the coreless motor having protective sleeves includes a stator assembly 4, a rotor assembly 3 and a motor housing 5. The stator assembly 4 is disposed in the motor housing 5 and defines an axially extending through-cavity. The rotor assembly 3 is rotatably disposed in the cavity. The motor housing 5 is fitted over an outer wall of the stator assembly 4, and opposite ends of the motor housing 5 extend beyond opposite ends of the stator assembly 4 in an axial direction. A stator protective sleeve 2 is disposed on an inner wall of the stator assembly 4, and opposite ends of the stator protective sleeve 2 extend beyond the opposite ends of the stator assembly 4 in the axial direction. Stator encapsulation layers 7 are disposed at the opposite ends of the stator assembly 4. The stator protective sleeve 2, the motor housing 5 and the stator encapsulation layers 7 hermetically isolate the stator assembly 4. The rotor assembly 3 includes a permanent magnet 32 and a rotor shaft 31, and the rotor shaft 31 extends through a center of the permanent magnet 32. A rotor protective sleeve 1 is disposed on an outer wall of the permanent magnet 32, and opposite ends of the rotor protective sleeve 1 extend beyond opposite ends of the permanent magnet 32 in the axial direction. Rotor encapsulation layers 6 are disposed at end portions of the permanent magnet 32. The rotor protective sleeve 1 and the rotor encapsulation layers 6 hermetically isolate the permanent magnet 32.

In some embodiments, an outer diameter φC1 of the stator protective sleeve 2 is smaller than an inner diameter φC of the stator assembly 4, and a minimum difference between the outer diameter φC1 of the stator protective sleeve 2 and the inner diameter φC of the stator assembly 4 is 0.02 mm, i.e., φC - φC1 = 0.02 mm. A maximum difference between the outer diameter φC1 of the stator protective sleeve 2 and the inner diameter φC of the stator assembly 4 is determined according to the size of the motor, as long as no friction occurs between the stator and the rotor. For example, when the outer diameter of the motor is less than 6 mm, the difference between the outer diameter φC1 of the stator protective sleeve 2 and the inner diameter φC of the stator assembly 4 may be in a range of 0.02 mm to 0.03 mm. The stator protective sleeve 2 is disposed coaxially with the stator assembly 4, such that a minimum clearance formed between the stator protective sleeve 2 and the stator assembly 4 is 0.01 mm. The stator protective sleeve 2 and the stator assembly 4 are bonded by an adhesive coated on an outer wall of the stator protective sleeve 2. A small clearance between the stator protective sleeve 2 and the stator assembly 4 facilitates nested coaxial bonding and installation of the two without occupying a large radial space, thereby avoiding an increase in motor size.

In some embodiments, a difference between a length D1 of the stator protective sleeve 2 and a length D of the stator assembly 4 is 1 mm to 2 mm, i.e., D1 - D = 1 mm to 2 mm. The stator assembly 4 includes a winding coil 41 and an iron core 42 arranged sequentially from inside to outside. In the axial direction, the winding coil 41 is usually disposed inside the iron core 42, and the length D of the stator assembly 4 generally refers to a length of the iron core 42. A distance between an end portion of the stator protective sleeve 2 and an end portion of the stator assembly 4 on the same side is 0.5 mm to 1 mm. The stator encapsulation layer 7 is disposed between the end portion of the stator protective sleeve 2 and the end portion of the stator assembly 4 on the same side. The stator encapsulation layers 7 are embedded and filled at both axial ends of the stator assembly 4, i.e., fully filling end faces of both axial ends of the stator assembly 4 in the axial direction to cover the entire area of the end faces, thereby ensuring the sealing effect and preventing delamination due to vibration during motor operation.

In some embodiments, distances between end portions of both ends of the stator protective sleeve 2 and end portions of the stator assembly 4 on the same side may be the same or different, that is, axial heights of the stator encapsulation layers 7 at both ends of the stator assembly may be the same or different, as long as a single-side potting height is greater than 0.3 mm.

In some embodiments, an inner diameter φA1 of the rotor protective sleeve 1 is larger than an outer diameter φA of the permanent magnet 32, and a minimum difference between the inner diameter φA1 of the rotor protective sleeve 1 and the outer diameter φA of the permanent magnet 32 is 0.02 mm, i.e., φA1 - φA = 0.02 mm. A maximum difference between the inner diameter φA1 of the rotor protective sleeve 1 and the outer diameter φA of the permanent magnet 32 is determined according to the size of the motor, as long as no friction occurs between the stator and the rotor. For example, when the outer diameter of the motor is less than 6 mm, the difference between the inner diameter φA1 of the rotor protective sleeve 1 and the outer diameter φA of the permanent magnet 32 may be in a range of 0.02 mm to 0.03 mm. The rotor protective sleeve 1 is disposed coaxially with the permanent magnet 32, such that a minimum clearance formed between the rotor protective sleeve 1 and the permanent magnet 32 is 0.01 mm. The rotor protective sleeve 1 and the permanent magnet 32 are bonded by an adhesive coated on an inner wall of the rotor protective sleeve 1. A small clearance between the rotor protective sleeve 1 and the rotor assembly 3 facilitates nested coaxial bonding and installation of the two without occupying a large radial space, thereby avoiding an increase in motor size.

In some embodiments, a difference between a length B1 of the rotor protective sleeve 1 and a length B of the permanent magnet 32 is 1 mm to 2 mm. A distance between an end portion of the rotor protective sleeve 1 and an end portion of the permanent magnet 32 on the same side is 0.5 mm to 1 mm. The rotor encapsulation layer 6 is disposed between the end portion of the rotor protective sleeve 1 and the end portion of the permanent magnet 32 on the same side. The rotor encapsulation layers 6 are embedded and filled at both axial ends of the permanent magnet 32, i.e., fully filling end faces of both axial ends of the permanent magnet 32 in the axial direction to cover the entire area of the end faces, thereby ensuring the sealing effect and preventing delamination due to vibration during motor operation.

In some embodiments, distances between end portions of both ends of the rotor protective sleeve 1 and end portions of the permanent magnet 32 on the same side may be the same or different, that is, axial heights of the rotor encapsulation layers 6 at both ends of the permanent magnet may be the same or different, as long as a single-side potting height is greater than 0.3 mm.

In some embodiments, an outer diameter of the stator assembly 4 is equal to an inner diameter of the motor housing 5, and the stator assembly 4 is connected to the motor housing 5 via a clearance fit. In specific implementation, the stator protective sleeve 2 may be first radially bonded and fixed to the stator assembly 4 with an adhesive, and then the stator assembly 4 with the stator protective sleeve 2 assembled thereon is positioned with the motor housing 5 by a positioning device, and then the stator assembly 4 and the motor housing 5 are cured and connected with an adhesive.

In some embodiments, the stator encapsulation layers 7 and the rotor encapsulation layers 6 are each formed by potting with an adhesive.

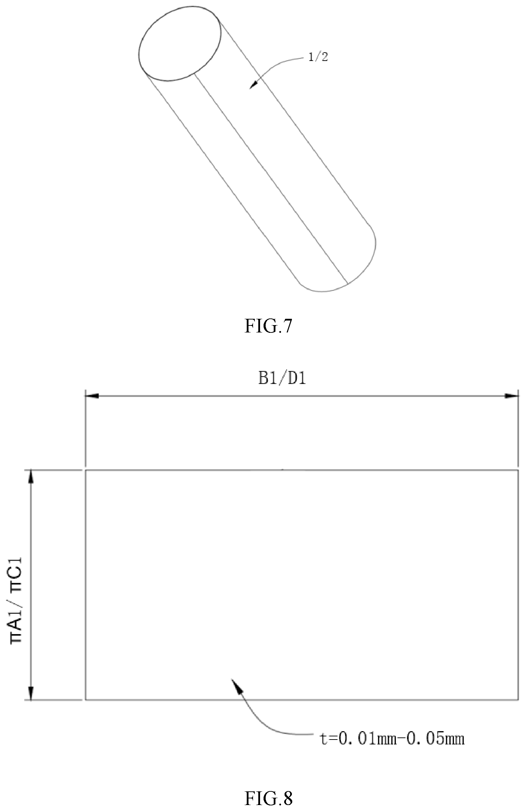

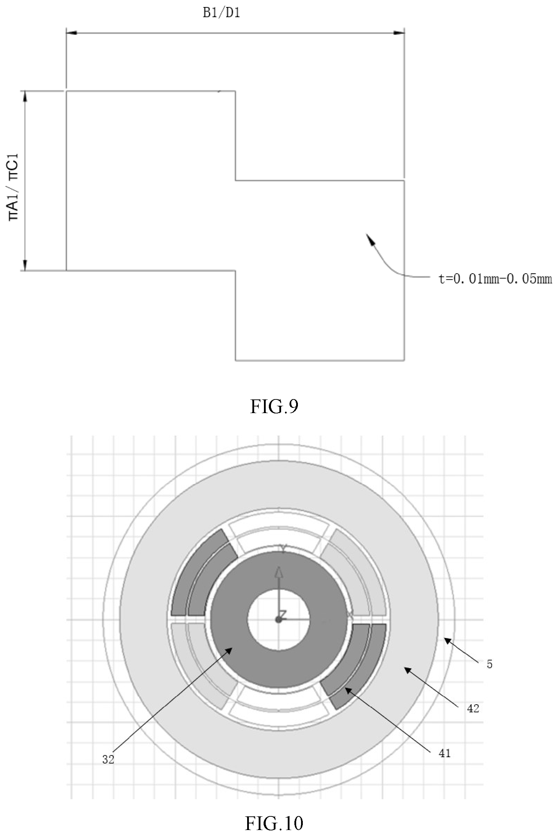

FIG. 7 is a schematic structural diagram illustrating a stator protective sleeve or a rotor protective sleeve according to an embodiment of the present application; FIG. 8 is a schematic structural diagram illustrating a thin metal sheet according to an embodiment of the present application; and FIG. 9 is a schematic structural diagram illustrating another thin metal sheet according to an embodiment of the present application.

Referring to FIGS. 7 to 9, the stator protective sleeve 2 and the rotor protective sleeve 1 each have a cylindrical tubular structure, and are each formed by welding a thin metal sheet. The thin metal sheet has a thickness t = 0.01 mm to 0.05 mm to ensure that only plastic deformation occurs. πA/πC represents a circumference of the rotor protective sleeve 1/the stator protective sleeve 2, and B1/D1 represents an axial length of the rotor protective sleeve 1/the stator protective sleeve 2. The shape of the thin metal sheet includes but is not limited to a rectangle, and other thin sheet structures that can be formed into a cylinder may also be used.

The thin metal sheets selected for the stator protective sleeve 2 and the rotor protective sleeve 1 need to meet biocompatibility requirements, and only undergo plastic deformation at a thickness of 0.01 mm to 0.05 mm, so as to be rolled into a cylindrical tubular structure. Preferably, the thin metal sheets have certain electrical conductivity to achieve the function of reducing spatial harmonics. Usually, the sleeves are formed by welding titanium alloy sheets, and the thin metal sheets may also be made of 316 stainless steel, 317 stainless steel, cobalt alloy, gold, silver, platinum, shape memory alloy and other materials.

In the prior art, other motors such as permanent magnet motors also use a permanent magnet protective sleeve fitted over the permanent magnet to achieve roundness positioning of surface-mounted permanent magnets and relative positioning of Hall components. The permanent magnet protective sleeve may be formed by plastic injection molding or metal machining to ensure positioning, and achieves certain magnetic circuit optimization when made of metal material. Compared with the permanent magnet protective sleeve, the rotor protective sleeve 1 in the embodiment of the present application is made of a biocompatible metal material and applied to a coreless motor. Since the motor is very small in size, the rotor protective sleeve 1 is required to have a small wall thickness (0.01 mm to 0.05 mm). At such wall thickness, the metal material tends to lose elastic resilience and undergo plastic deformation. Therefore, it cannot be formed by machining in terms of manufacturing process. Instead, a thin metal sheet made of biocompatible metal material needs to be rolled into a cylindrical structure and welded into shape, and disposed outside the rotor to shield and isolate non-biocompatible materials, with relatively high rigidity and strength.

In some embodiments, a Flexible Printed Circuit (FPC), a Printed Circuit Board (PCB), a bracket or the like is disposed at an end portion of the stator assembly 4 to connect the winding coil 41 of the stator assembly 4 with an external power line, as well as other auxiliary parts. The parts disposed at the end portion of the stator assembly 4 are sealed together by the stator encapsulation layer 7 after being fixed.

In some embodiments, auxiliary parts such as a balance ring or a limit retaining ring are disposed at axial end portions of the rotor assembly 3. The limit retaining ring or the balance ring is a part for assisting rotor positioning or meeting dynamic balance requirements, and may be disposed at both ends of the permanent magnet. The auxiliary parts disposed at the end portion of the rotor assembly 3 are sealed together by the rotor encapsulation layer 6 after being fixed. After adding the auxiliary parts, since end faces of the auxiliary parts are attached to the permanent magnet 32, the adhesive volume of the rotor encapsulation layer 6 on one side may be reduced.

An embodiment of the present application further provides a potting method for a coreless motor having protective sleeves. The coreless motor having protective sleeves includes a stator assembly 4, a rotor assembly 3 and a motor housing 5. The stator assembly 4 is disposed in the motor housing 5 and defines an axially extending through-cavity. The rotor assembly 3 is rotatably disposed in the cavity. The rotor assembly 3 includes a permanent magnet 32 and a rotor shaft 31, and the rotor shaft 31 extends through a center of the permanent magnet 32. The method includes the following steps:
the stator assembly 4 and a stator protective sleeve 2 are provided, the stator protective sleeve 2 is fitted over an inner wall of the stator assembly 4, and opposite ends of the stator protective sleeve 2 are controlled to extend axially beyond opposite ends of the stator assembly 4, with the opposite ends of the stator protective sleeve 2 being equidistant from the opposite ends of the stator assembly 4 in the axial direction, respectively;
the motor housing 5 is provided, the motor housing 5 is fitted over an outer wall of the stator assembly 4 with the stator protective sleeve 2 mounted thereon, and opposite ends of the motor housing 5 are controlled to extend axially beyond the opposite ends of the stator assembly 4, such that a stator potting space is defined by an outer wall of the stator protective sleeve 2, an inner wall of the motor housing 5, an end face of the stator assembly 4 and an end face of the stator protective sleeve 2; at this time, the stator protective sleeve 2 is bonded to the winding coil 41 of the stator assembly 4 with an adhesive, the stator assembly 4 is cured and bonded to the motor housing 5 with an adhesive, and the radial bonding is completed; a groove is formed between the outer wall of the stator protective sleeve 2 and the inner wall of the motor housing 5 in the axial direction, and the stator potting space to be filled with adhesive is formed by the groove; an axial height of the adhesive is limited by the end face of the stator protective sleeve 2, and a bottom surface is formed by end faces of the iron core 42 and the winding coil 41 of the stator assembly 4;
an adhesive is dispensed into the stator potting space to form a stator encapsulation layer 7, so as to hermetically isolate the stator assembly 4; air in the stator potting space is gradually discharged through dispensing to avoid air bubbles from affecting the sealing effect;
the rotor assembly 3 and a rotor protective sleeve 1 are provided, the rotor protective sleeve 1 is fitted over an outer wall of the permanent magnet 32, and opposite ends of the rotor protective sleeve 1 are controlled to extend axially beyond opposite ends of the permanent magnet 32, with the opposite ends of the rotor protective sleeve 1 being equidistant from the opposite ends of the permanent magnet 32 in the axial direction, respectively, such that a rotor potting space is defined by an outer wall of the rotor shaft 31, an inner wall of the rotor protective sleeve 1, an end face of the permanent magnet 32 and an end face of the rotor protective sleeve 1; at this time, the rotor shaft 31 is radially connected to the permanent magnet 32, the rotor protective sleeve 1 is radially bonded to the permanent magnet 32, and the radial bonding is completed; a groove is formed between the outer wall of the rotor shaft 31 and the inner wall of the rotor protective sleeve 1 in the axial direction, and the rotor potting space to be filled with adhesive is formed by the groove; an axial height of the adhesive is limited by the end face of the rotor protective sleeve 1, and a bottom surface is formed by the end face of the permanent magnet 32; and
an adhesive is dispensed into the rotor potting space to form a rotor encapsulation layer 6, so as to hermetically isolate the permanent magnet 32; air in the rotor potting space is gradually discharged through dispensing to avoid air bubbles from affecting the sealing effect.

After potting is completed, the coreless motor is assembled: the rotor assembly 3 with the rotor protective sleeve 1 mounted and potted is installed into the cavity, a front-end cover 8 and a rear end cover 9 for fixing the rotor assembly 3 are installed at both ends of the motor housing 5, and a connection cover 10 for introducing an external power line is installed on the rear end cover 9 to complete the assembly.

In specific implementation, an outer diameter φC1 of the stator protective sleeve 2 is smaller than an inner diameter φC of the stator assembly 4, the stator protective sleeve 2 is disposed coaxially with the stator assembly 4, such that a minimum difference between the outer diameter φC1 of the stator protective sleeve 2 and the inner diameter φC of the stator assembly 4 is 0.02 mm, i.e., φC - φC1 = 0.02 mm, and a minimum clearance formed between the stator protective sleeve 2 and the stator assembly 4 is 0.01 mm. The potting method includes the following steps:
an outer wall of the stator protective sleeve 2 is coated with an adhesive; and
the stator protective sleeve 2 is fitted over the inner wall of the stator assembly 4, so as to bond the stator protective sleeve 2 and the stator assembly 4 into one piece.

In specific implementation, an inner diameter φA1 of the rotor protective sleeve 1 is larger than an outer diameter φA of the permanent magnet 32, the rotor protective sleeve 1 is disposed coaxially with the permanent magnet 32, such that a minimum difference between the inner diameter φA1 of the rotor protective sleeve 1 and the outer diameter φA of the permanent magnet 32 is 0.02 mm, i.e., φA1 - φA = 0.02 mm, and a minimum clearance formed between the rotor protective sleeve 1 and the permanent magnet 32 is 0.01 mm. The potting method includes the following steps:
an inner wall of the rotor protective sleeve 1 is coated with an adhesive; and
the rotor protective sleeve 1 is fitted over the outer wall of the permanent magnet 32, so as to bond the rotor protective sleeve 1 and the rotor assembly 3 into one piece.

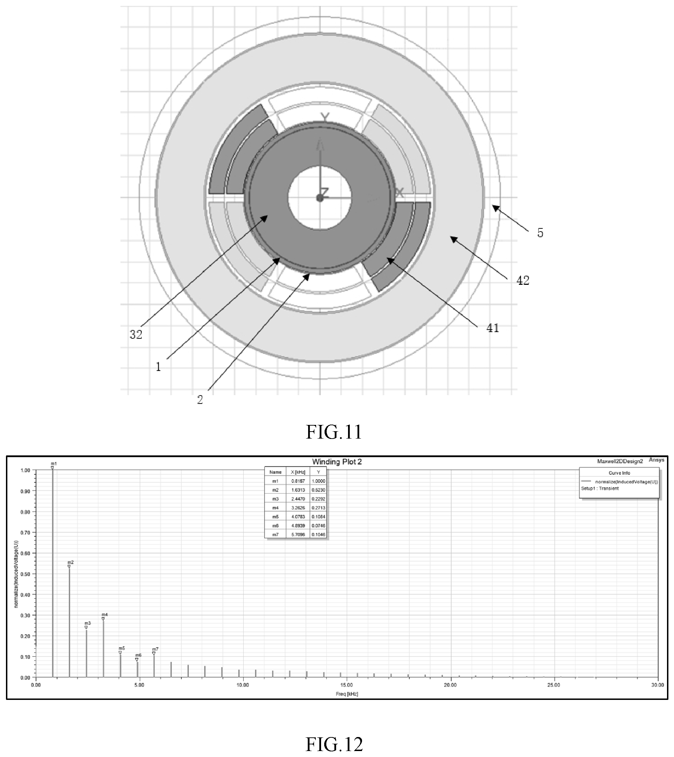

FIG. 10 is a schematic model diagram illustrating a coreless motor without a protective sleeve in the prior art; and FIG. 11 is a schematic model diagram illustrating a coreless motor having protective sleeves according to an embodiment of the present application.

Referring to FIGS. 10 and 11, the radial volume of the coreless motor having protective sleeves is substantially the same as that of the coreless motor without a protective sleeve, indicating that the arrangement of the stator protective sleeve 2 and the rotor protective sleeve 1 has substantially no effect on the volume of the coreless motor.

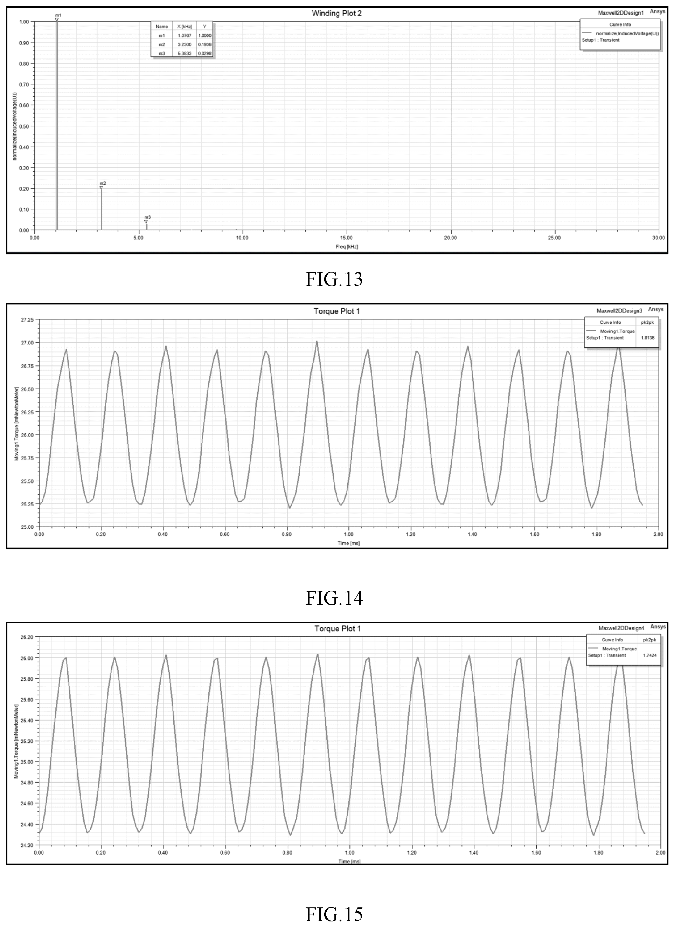

FIG. 12 is an FFT diagram illustrating back EMF of a coreless motor without a protective sleeve in the prior art; and FIG. 13 is an FFT diagram illustrating back EMF of a coreless motor having protective sleeves according to an embodiment of the present application.

Referring to FIGS. 12 and 13, the abscissa represents frequency in KHz, and the ordinate represents back EMF. The number of harmonics decomposed by FFT of no-load back EMF of the coreless motor having protective sleeves is greatly reduced compared with that of the coreless motor without a protective sleeve. Reducing the FFT decomposition harmonics of no-load back EMF means reducing the fundamental component of no-load back EMF, thereby improving the output capability of the motor, and reducing harmonics also greatly improves noise and vibration.

FIG. 14 is a torque diagram illustrating a coreless motor without a protective sleeve in the prior art; and FIG. 15 is a torque diagram illustrating a coreless motor having protective sleeves according to an embodiment of the present application.

Referring to FIGS. 14 and 15, the abscissa represents time in ms, and the ordinate represents torque. The torque ripple of the coreless motor having protective sleeves is 1.74 mN·m, and the torque ripple of the coreless motor without a protective sleeve is 1.81 mN·m, so the torque ripple of the coreless motor having protective sleeves is reduced by about 4%. With the reduction of torque ripple range, the operation stability of the motor is improved; the reduction of cogging torque reduces the vibration of the motor.

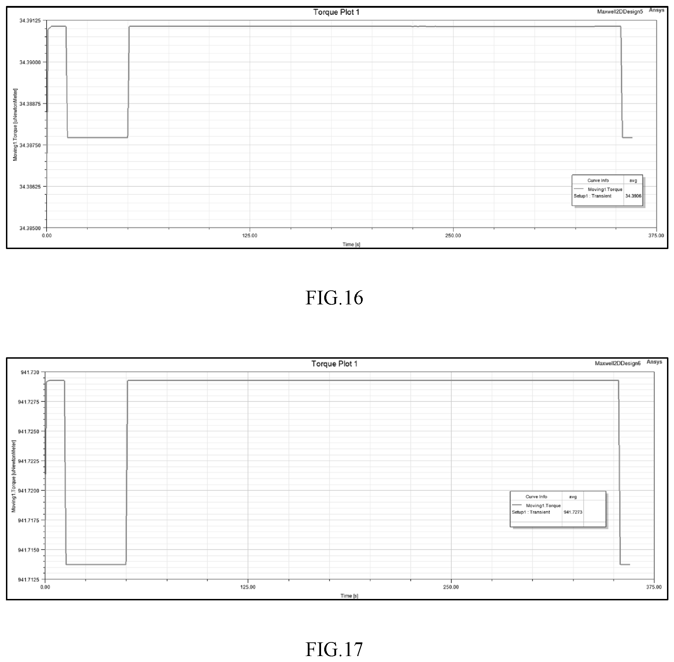

FIG. 16 is a cogging torque diagram illustrating a coreless motor without a protective sleeve in the prior art; and FIG. 17 is a torque diagram illustrating a coreless motor having protective sleeves according to an embodiment of the present application.

Referring to FIGS. 16 and 17, the abscissa represents rotation angle, and the ordinate represents cogging torque. The cogging torque of the coreless motor having protective sleeves is 0.03 mN, and the cogging torque of the coreless motor without a protective sleeve is 0.9 mN. It can be seen that the cogging torque of the coreless motor having protective sleeves is significantly reduced.

The coreless motor having protective sleeves can effectively filter temporal and spatial harmonics in the stator magnetic field, reduce eddy current loss on the rotor surface, reduce heat generation of the rotor, and improve motor efficiency.

In summary, in the coreless motor having protective sleeves and the method of potting same according to the present application, protective sleeves are used to protect the stator assembly 4 and the rotor assembly 3, and sealing and isolation are realized by dispensing adhesive at end portions to form encapsulation layers, thereby achieving reliable protection for non-biocompatible materials of the motor. Especially when water enters the motor, the encapsulation layers are not easy to delaminate under water pressure scouring, with high reliability.

Further, the protective sleeves of the present application are processed from thin metal sheets, usually titanium alloy. Titanium alloy has good biocompatibility, high strength and stable structure, which is conducive to providing stable and reliable protection. Titanium alloy has low electrical conductivity, which can effectively filter temporal and spatial harmonics in the stator magnetic field, reduce eddy current loss on the rotor surface, reduce heat generation of the rotor, improve motor efficiency, and ensure the safety of motor operation. The welding process is simple and easy to manufacture, and the small thickness of the thin metal sheet does not affect the overall size of the motor.

Further, the protective sleeves of the present application extend beyond both ends of the stator assembly 4 or the permanent magnet 32 of the rotor assembly 3 to form potting spaces, which facilitates dispensing of the encapsulation layers to achieve sealing, and prevents the encapsulation layers from delaminating due to vibration during motor operation.

Finally, it should be noted that the axial direction, radial direction and circumferential direction in the embodiments of the present application refer to the axial direction, radial direction and circumferential direction of the rotor assembly 3, respectively.

Although specific embodiments have been described above, these embodiments are not intended to limit the scope of the present application, even if only a single embodiment is described with respect to a specific feature. The feature examples provided in the present application are intended to be illustrative, not limiting, unless otherwise stated. In specific implementation, according to actual requirements and under technically feasible conditions, the technical features of one or more dependent claims may be combined with the technical features of the independent claim, and the technical features from corresponding independent claims may be combined in any appropriate manner, not only by the specific combinations recited in the claims.

Although the present application has been disclosed as above, the present application is not limited thereto. Any person skilled in the art can make various changes and modifications without departing from the spirit and scope of the present application. Therefore, the protection scope of the present application shall be subject to the scope defined by the claims.

## Claims

1. A coreless motor having protective sleeves, comprising a stator assembly, a rotor assembly and a motor housing; the rotor assembly being disposed in the motor housing and defining an axially extending through-cavity, the rotor assembly being rotatably disposed in the cavity, wherein the motor housing is fitted over an outer wall of the stator assembly, and opposite ends of the motor housing extend axially beyond opposite ends of the stator assembly; a stator protective sleeve is disposed on an inner wall of the stator assembly, and opposite ends of the stator protective sleeve extend axially beyond the opposite ends of the stator assembly; stator encapsulation layers are disposed at the opposite ends of the stator assembly, and the stator protective sleeve, the motor housing and the stator encapsulation layers hermetically isolate the stator assembly; the rotor assembly comprises a permanent magnet and a rotor shaft, the rotor shaft extends through a center of the permanent magnet; a rotor protective sleeve is disposed on an outer wall of the permanent magnet, and opposite ends of the rotor protective sleeve extend axially beyond opposite ends of the permanent magnet; rotor encapsulation layers are disposed at end portions of the permanent magnet, and the rotor protective sleeve and the rotor encapsulation layers hermetically isolate the permanent magnet.

2. The coreless motor having protective sleeves according to claim 1, wherein the stator protective sleeve and the rotor protective sleeve are each made of a biocompatible metal material, and the biocompatible metal material comprises 316 stainless steel, 317 stainless steel, titanium alloy, cobalt alloy, gold, silver, platinum, and shape memory alloy.

3. The coreless motor having protective sleeves according to claim 2, wherein the stator protective sleeve and the rotor protective sleeve each have a cylindrical tubular structure, and are each formed by welding a thin metal sheet made of the biocompatible metal material, and the thin metal sheet has a thickness of 0.01 mm to 0.05 mm.

4. The coreless motor having protective sleeves according to claim 1, wherein the stator encapsulation layers and the rotor encapsulation layers are each formed by potting with an adhesive.

5. The coreless motor having protective sleeves according to claim 1, wherein an outer diameter of the stator protective sleeve is smaller than an inner diameter of the stator assembly, a minimum difference between the outer diameter of the stator protective sleeve and the inner diameter of the stator assembly is 0.02 mm; the stator protective sleeve is disposed coaxially with the stator assembly, such that a minimum clearance formed between the stator protective sleeve and the stator assembly is 0.01 mm; and the stator protective sleeve and the stator assembly are bonded by an adhesive coated on an outer wall of the stator protective sleeve.

6. The coreless motor having protective sleeves according to claim 1, wherein a difference between a length of the stator protective sleeve and a length of the stator assembly is 1 mm to 2 mm; a distance between an end portion of the stator protective sleeve and an end portion of the stator assembly on a same side is 0.5 mm to 1 mm; and the stator encapsulation layer is disposed between the end portion of the stator protective sleeve and the end portion of the stator assembly on the same side.

7. The coreless motor having protective sleeves according to claim 1, wherein an inner diameter of the rotor protective sleeve is larger than an outer diameter of the permanent magnet, a minimum difference between the inner diameter of the rotor protective sleeve and the outer diameter of the permanent magnet is 0.02 mm; the rotor protective sleeve is disposed coaxially with the permanent magnet, such that a minimum clearance formed between the rotor protective sleeve and the permanent magnet is 0.01 mm; and the rotor protective sleeve and the permanent magnet are bonded by an adhesive coated on an inner wall of the rotor protective sleeve.

8. The coreless motor having protective sleeves according to claim 1, wherein a difference between a length of the rotor protective sleeve and a length of the permanent magnet is 1 mm to 2 mm; a distance between an end portion of the rotor protective sleeve and an end portion of the permanent magnet on a same side is 0.5 mm to 1 mm; and the rotor encapsulation layer is disposed between the end portion of the rotor protective sleeve and the end portion of the permanent magnet on the same side.

9. The coreless motor having protective sleeves according to claim 1, wherein an outer diameter of the stator assembly is equal to an inner diameter of the motor housing, and the stator assembly is connected to the motor housing via a clearance fit.

10. A method of potting a coreless motor having protective sleeves according to any one of claims 1 to 9, the coreless motor having protective sleeves comprising a stator assembly, a rotor assembly and a motor housing; the stator assembly being disposed in the motor housing and defining an axially extending through-cavity, the rotor assembly being rotatably inserted through the cavity, and the rotor assembly comprising a permanent magnet and a rotor shaft, the rotor shaft extending through a center of the permanent magnet; wherein the method comprises the steps of:
providing the stator assembly and a stator protective sleeve, fitting the stator protective sleeve over an inner wall of the stator assembly, and controlling opposite ends of the stator protective sleeve to extend axially beyond opposite ends of the stator assembly, with the opposite ends of the stator protective sleeve being equidistant from the opposite ends of the stator assembly in the axial direction, respectively;
providing the motor housing, fitting the motor housing over an outer wall of the stator assembly with the stator protective sleeve mounted thereon, and controlling opposite ends of the motor housing to extend axially beyond the opposite ends of the stator assembly, such that a stator potting space is defined by an outer wall of the stator protective sleeve, an inner wall of the motor housing, an end face of the stator assembly and an end face of the stator protective sleeve;
dispensing an adhesive into the stator potting space to form a stator encapsulation layer, so as to hermetically isolate the stator assembly;
providing the rotor assembly and a rotor protective sleeve, fitting the rotor protective sleeve over an outer wall of the permanent magnet, and controlling opposite ends of the rotor protective sleeve to extend axially beyond opposite ends of the permanent magnet, with the opposite ends of the rotor protective sleeve being equidistant from the opposite ends of the permanent magnet in the axial direction, respectively, such that a rotor potting space is defined by an outer wall of the rotor shaft, an inner wall of the rotor protective sleeve, an end face of the permanent magnet and an end face of the rotor protective sleeve; and
dispensing an adhesive into the rotor potting space to form a rotor encapsulation layer, so as to hermetically isolate the permanent magnet.

11. The method of potting a coreless motor having protective sleeves according to claim 10, wherein an outer diameter of the stator protective sleeve is smaller than an inner diameter of the stator assembly, the stator protective sleeve is disposed coaxially with the stator assembly, such that a minimum difference between the outer diameter of the stator protective sleeve and the inner diameter of the stator assembly is 0.02 mm, and a minimum clearance formed between the stator protective sleeve and the stator assembly is 0.01 mm; the method further comprising the steps of:
coating an outer wall of the stator protective sleeve with an adhesive; and
fitting the stator protective sleeve over the inner wall of the stator assembly, so as to bond the stator protective sleeve and the stator assembly into one piece.

12. The method of potting a coreless motor having protective sleeves according to claim 10, wherein an inner diameter of the rotor protective sleeve is larger than an outer diameter of the permanent magnet, the rotor protective sleeve is disposed coaxially with the permanent magnet, such that a minimum difference between the inner diameter of the rotor protective sleeve and the outer diameter of the permanent magnet is 0.02 mm, and a minimum clearance formed between the rotor protective sleeve and the permanent magnet is 0.01 mm; the method further comprising the steps of:
coating an inner wall of the rotor protective sleeve with an adhesive; and
fitting the rotor protective sleeve over the outer wall of the permanent magnet, so as to bond the rotor protective sleeve and the rotor assembly into one piece.
